(19) Europäisches Patentamt
European Patent Office
Office européen des brevets

(11) Publication number: **0 175 485**
**A2**

# (12) EUROPEAN PATENT APPLICATION

(21) Application number: **85305893.1**

(22) Date of filing: **19.08.85**

(51) Int. Cl.⁴: **C 11 D 17/00**, A 61 K 7/08, A 61 K 7/50

(30) Priority: **21.08.84 GB 8421196**

(43) Date of publication of application: **26.03.86 Bulletin 86/13**

(84) Designated Contracting States: **AT BE CH DE FR GB IT LI LU NL SE**

(71) Applicant: **RECKITT & COLMAN PRODUCTS LIMITED, P.O. Box 26 1-17 Burlington Lane, London W4 2RW (GB)**

(72) Inventor: **Cook, Robert Victor, 11 Greenleas, Aston on Trent Derby, DE7 2UQ (GB)**
Inventor: **Pool, Hazel, 211 Lambourn Drive, Allestree Derby, DE3 2UR (GB)**

(74) Representative: **Allard, Susan Joyce et al, BOULT, WADE & TENNANT 27 Furnival street, London EC4A 1PQ (GB)**

(54) **Shampoo compositions.**

(57) Hair or body shampoo having an upper aqueous layer and a lower aqueous layer which are temporily dispersible one in the other upon agitation of the composition and wherein each layer is miscible with water in substantially all proportions. The upper has dissolved therein at least one detergent. The lower layer contains a sequestering agent such as certain organophosphates, aminocarboxylic acids and/or pollyhydroxycarboxylic acids containing 5 to 7 carbon atoms. The compositions have cleansing properties and impart a conditioning effect.

EP 0 175 485 A2

SHAMPOO COMPOSITIONS

This invention relates to aqueous cleansing compositions having at least two distinct water-miscible liquid phases.

Multiphase liquid cleansing compositions containing water for bath or shower use and for cleansing hair have been proposed.

Some of the prior art compositions have relied upon the inclusion of a hydrocarbon oil or similar oily component which is substantially immiscible with water and consequently forms a separate layer after a simple mixture thereof with water has been allowed to stand undisturbed.

In other prior art compositions, for example those disclosed in British specification N° 1247189, substantial concentrations of hydroxylic solvents such as the lower aliphatic alcohols have been included. Whilst such hydroxylic compounds are themselves miscible with water in more or less all proportions, in the presence of aqueous surfactants solutions containing water-soluble inorganic salts a "salting out" effect occurs which results in a multiphase composition from which two distinct liquid layers separate when a previously shaken mixture is allowed to stand undisturbed.

Three phase liquid mixtures which are a combination of the above that is having two aqueous phases achieved by "salting out" with a supernatant oil layer have been proposed. Furthermore, three phase liquid mixtures have also been prepared by admixing a single aqueous phase with hydrocarbon oils and certain silicones which latter are

immiscible both with hydrocarbon oils and water.

The first mentioned group of prior art compositions suffers from the disadvantages firstly that both phases are not miscible with water in all proportions; secondly, an excess of oil in a shampoo can be undesirable especially for people with naturally oil-rich heads of hair; further, oil tends to be deposited on the hair leading to unsightly, greasy hair that is unattractive even on hair that is naturally not oil rich. Even for those having relatively "dry" hair the path to improved manageability is not necessarily via the mere addition of oils to the hair.

The second mentioned group of prior art compositions in which both phases are miscible with water in substantially all proportions, suffer from the disadvantages firstly, of the undesirable smell of alcohol that needs to be masked and which can lead to unwarranted formula modifications detrimental to performance; secondly, the inclusion of much alcohol tends to have a denaturing effect, even on naturally greasy hair, by stripping of the natural oils from the hair shaft. It will be appreciated that for those with naturally "dry" hair such stripping of meagre natural oils is highly undesirable; thirdly, such alcohol-rich compositions tend to have low viscosities, leading to inconvenience in use.

The third mentioned group of prior art compositions suffers from the disadvantages to be expected from a combination of the two types of composition. Moreover to add a further water-immiscible material does not mitigate the ill effects of an oily composition.

In our copending British Patent Application No.

8402686, we have proposed to mitigate the abovementioned disadvantages by combining aqueous surfactants having a high foaming potential with an aqueous solution of sodium hexametaphosphate to produce a two layer composition in which both layers are miscible with water in all proportions. An effective two-layer shampoo composition, for use inter alia on the hair, is thus achieved without recourse to the use of oils or hydroxylic solvents. It has to be observed that minimal amounts of lower aliphatic alcohols, significantly below 2% w/w of the total composition are admitted in the compositions the subject of the abovementioned British Patent Application, for example to assist the solubilisation of cosmetic components such as perfumes and other additaments of an oily nature.

U.S.-A-3313735 discloses the use of 0.2-5% of phosphono-compounds, in particular alkylphosphono-acetic acids in conjunction with 10-30% of certain non-soap anionic surfactants which are all dissolved in water to form a clear homogeneous solution which is useful as a hair conditioning shampoo. The specification does not disclose multiphase compositions.

It is known that the condensed phosphates, such as sodium hexametaphosphate, undergo slow hydrolysis in an aqueous medium. Such hydrolysis can, in some cases, lead to unsightly products which may not be attractive to the ultimate user of a shampoo composition notwithstanding that its efficacy as a shampoo is thereby unimpaired. Since elevated temperatures such as may be encountered for example during storage, increase the rate of hydrolysis, the defect

described is considered undesirable in a cosmetic preparation.

We have now found that liquid multiphase cleansing compositions, the separate liquid phases of which are miscible with water, and which are satisfactory for bath or shower use and as hair shampoos may be prepared without recourse to the use of the abovementioned oils, hydroxylic solvents or sodium hexametaphosphate.

Accordingly the present invention provides liquid cleansing compositions comprising water; at least one surfactant; and at least one sequestering agent, the relative concentrations of the components being so chosen that the composition has at least two distinct layers which are temporarily interdispersible upon agitation and wherein each layer is miscible with water in substantially all proportions.

The concentration of surfactant is preferably up to 50% by weight of the total composition.

The concentration of sequestering agent is preferably up to 30% by weight of the total composition.

Preferably the surfactant comprises at least one anionic, amphoteric or nonionic surfactant or a mixture thereof.

More preferably the surfactant comprises at least one anionic surfactant and at least one surfactant other than an anionic surfactant.

The anionic surfactant is preferably a blend of anionic surface active agents. Generally, the anionic surfactant or blend thereof will be chosen so as to yield a

highly foamant composition be it for bath/shower use or as a hair shampoo. Whether a single anionic or a blend thereof is used, it is so chosen that it has a gentle action on the hair when the composition according to the invention is to be used as a hair shampoo. Furthermore, for use as hair shampoos those surfactants known to have condition-enhancing effects in use on the hair will be chosen.

Individual surfactants include soaps, for example sodium laureth-13 carboxylate and sodium trideceth-7 carboxylate, water-soluble salts of organic sulphuric reaction products having in their molecular structure an alkyl or alkylene group containing from about 8 to about 18 carbon atoms and a radical selected from the group consisting of sulphonic acid and sulphuric acid ester groups. Important examples of this group of anionic surfactants are sodium and potassium alkyl sulphates in which the alkyl group is similar to or the same as may be found in tallow or coconut oil reduction products; sodium or potassium alkyl benzene sulphonates which contain alkyl groups with about 9 to about 15 carbon atoms; as well as the ethoxylated derivatives of the above materials having up to about 10 moles of ethylene oxide per molecule; sodium alkylglycerylether sulphonates in which the alkyl groups are as obtained from alcohols derived from tallow or coconut oil; and acyl sarcosinates including about 10 to about 18 carbon atoms in the alkyl chain.

Preferred surfactants are mono-, di- and triethanolamine and sodium salts of alkyl sulphate in which the alkyl chain contains about 12 carbon atoms, sodium and

triethanolamine salts of the sulphated or sulphonated condensation product of 1 mole coconut fatty alcohol with up to 3 moles ethylene oxide.

Other anionic surfactants that may be employed include sulphosuccinates, such as sodium pareth-25 sulphosuccinate.

It will be appreciated by those versed in the art that with some compositions the anionic surfactant may be replaced at least in part by an amphoteric surfactant. Suitable examples of such are certain glycinates such as cocoamphocarboxyglycinate, cocoamphoglycinate, AMPHOLAK XCG/3 (Trade Mark) ex AMPHOTERICS INTERNATIONAL, which is a blend of cocoamphocarboxyglycinate and a complex N-cocoamine carboxylic acid of relatively low molecular weight between 400 and 800, in the ratio 85:15 by weight; and TEA-PEG-3 cocoamide sulphate.

The surfactant other than the anionic surfactant, herein referred to as the other surfactant, preferably includes a nonionic surfactant or an amphoteric surfactant or a blend thereof, that is mixtures of nonionic surfactants and mixtures of amphoteric surfactants. Furthermore blends of nonionic surfactants with amphoteric surfactants may be employed to advantage. Similarly, certain cationic surfactants may be included in some blends.

Typically, the following surfactants fall into this category, cocoamide DEA, PEG-6 cocoamide, oleamide DEA, linoleamide DEA, lauramide MIPA, PEG-120 methyl glucose dioleate, polyoxyethylene propylene glycol dioleate, PEG-20 glyceryl laurate, PEG-20 glyceryl stearate, PEG-20 glyceryl oleate, polyethylene glycol distearates of molecular weight

from about 1000 to about 10000 also similar phosphate esters including PPG-5-ceteth-10 phosphate, trilaureth-4 phosphate; betaines, such as cocoamidopropyl betaine, lauryl betaine; cocoamidopropyl dimethylamine betaine; amine oxides such as stearamine oxide, cocoamidopropylamine oxide, N-myristyl-dimethylamine oxide, PEG-lauramine oxide, dihydroxy-ethyltallowamine oxide; some sultaines such as cocoamidopropyl hydroxysultaine; some cationic surfactants such as quaternium 70, guar hydroxypropyltrimonium chloride, polyquaternium 4.

The sequestering agents used in the compositions of the present invention may be in the form of free acids or normal salts thereof. Preferably the sequestering agent is present in the form of a normal salt, that is to say a salt in which any free acid groups are electrically neutralised by a suitable alkali metal ion or a suitable organic base. A blend of sequestering agents may also be employed.

Preferably the sequestering agent provides a complexing anion which is strong enough for a 0.01M solution thereof to be in equilibrium with less than $10^{-3}$ mole of calcium ion.

Preferably, the materials used in compositions of the present invention and referred to herein as the sequestering agent fall into one of the following categories of compounds:- organophosphonic acids and their salts; aminocarboxylic acids and their salts and mixtures thereof, and optionally including polyhydroxycarboxylic acids and their salts; and/or inorganic salts such as sodium sesquicarbonate.

The sequestering agents have in common the feature of

chemical stability to hydrolysis under the conditions expected to be encountered by a cleansing composition, which distinguishes them as a group from sodium hexametaphosphate. Moreover it has been found that the sequestering agents when included in the compositions of the present invention for washing hair exhibit good hair conditioning properties, that is they at least contribute to the post-shampooing manageability, feel and appearance of the hair.

Accordingly, in a particular aspect the present invention provides a liquid hair shampoo composition comprising water; at least one anionic surfactant; at least one surfactant other than an anionic surfactant; and at least one sequestering agent selected from:-

(i) organophosphonates of general formula (I)

$$R^1\text{-}N\text{-}(R)_2 \qquad (I)$$

wherein $R = CH_2PO_3M_2$, M represents hydrogen, or a cosmetically acceptable organic and/or inorganic base; and $R^1$ is a straight or branched chain alkyl group which may contain an odd or even number of carbon atoms up to 14 in number, a hydroxyalkyl group containing from 2 to 6 carbon atoms, or a group

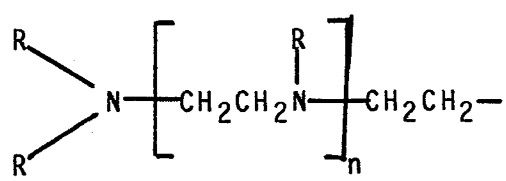

in which n = 0, 1, 2, 3 or 4; or $R^1$ = R: or

(ii) aminocarboxylic acids of general formula (II)

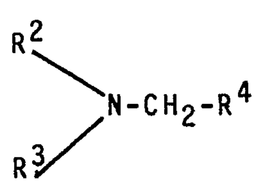

(II)

wherein $R^2 = H$; $-CH_2CH_2OH$ or $-CH_2COOM$;

$R^3 = -CH_2CH_2OH$, $-CH_2COOM$ or 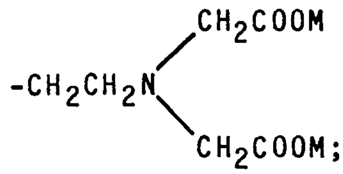

$R^4 = -COOM$ or 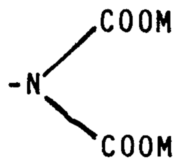

where M is as defined above: or mixtures thereof optionally containing:-

(iii) polyhydroxycarboxylic acids containing 5 to 7 carbon atoms, optionally in a ring, and more than two hydroxyl groups per molecule or their salts with cosmetically acceptable organic and/or inorganic bases: and/or (iv) sodium sesquicarbonate, the relative concentrations of the components being so chosen that the composition has at least two distinct layers which are temporarily interdispersible upon agitation and wherein each layer is miscible with water in substantially all proportions.

The compositions of the present invention will usually have 2 distinct layers, however the invention encompasses the possibility of having 3 or even 4 layers each of which is miscible with water in substantially all proportions.

The separate layers in the compositions of the present invention are preferably clear and bright. In a composition having only two layers, an upper layer, largely containing dissolved surfactant and a lower layer largely containing dissolved sequestering agent it is especially preferable to achieve clear, bright phases. The clarity of the layers

depends in part on the nature of the components, their purity and concentration.

The relative proportions of the selected components in the composition are determined by the overriding requirement for a multiphase composition.

Broadly, the maximum concentration of sequestering agent will be 25% by weight of the composition. The maximum total surfactant concentration will be 40% by weight of the composition. The maximum concentration of anionic surfactant will be 25% by weight of the composition.

Preferred ranges of concentrations based by weight of the final composition are 5 to 15% anionic surfactant or blend thereof, up to 15% of other surfactant or blend thereof, up to 15% of sequestering agent and more than 60% of water.

More preferably the concentration of sequestering agent is from 2-15%. The amount of sequestering agent is generally towards the low end of this range.

The proportion of water is preferably below 85%.

Liquid shampoos generally contain more than about 60% of water. Below that value they tend to be of a pasty consistency. At a concentration greater than 85% by weight of water, the amount of active material per unit dose may sometimes be insufficient. However, liquid shampoos with over 90% water occur in the market place at a price attractive to some consumers. It therefore has to be observed that some of the compositions of the present invention can still retain their multiphase state at such dilutions.

The compositions of the present invention are extremely complex because the materials used are generally industrial products which rarely contain a single substance. Nevertheless it has been found that an informative curve results from plotting relative concentrations in water of the three main components anionic surfactant, other surfactant and sequestering agent. By plotting such curves at given concentrations of water in the composition a good idea of the three dimensional phase diagram at a given temperature may be had.

The invention is further illustrated in the accompanying drawings, where concentrations are expressed as ratios from which absolute concentrations in terms of percentage by weight of the total composition may be determined in conjunction with the indicated water content which is fixed for each phase diagram, in which:-

Figure 1 is a plot on triangular coordinates of compositions which contain 60% water with various concentrations of triethanolamine lauryl sulphate as anionic surfactant, coconut diethanolamide as nonionic surfactant and the sodium salt of nitrilo tris (methylene phosphonic acid) as sequestering agent. The right upright side of the triangle represents increasing concentration of nonionic surfactant with maximum coconut diethanolamide at the apex of the triangle; the left upright side of the triangle represents increasing concentration of anionic surfactant with maximum triethanolamine lauryl sulphate at the left extremity of the base; the base of the triangle represents increasing concentration of sequestering agent with maximum

sodium nitrilo tris (methylene phosphonate) at the right; those compositions exhibiting a multiplicity of phases lie to the right of the boundary curve;

Figure 2 is a similar plot for a concentration 70% of water;

Figure 3 is a similar plot for a concentration 80% of water; and

Figure 4 is a similar plot for a concentration 90% of water;

Since, nominally, water represents an upright edge of a tetrahedron resting on its base, the size of these phase diagrams diminishes as the concentration of water increases.

It can be visualised from this collection of phase diagrams that the shape of the whole boundary surface is reminiscent of a partly filled sail.

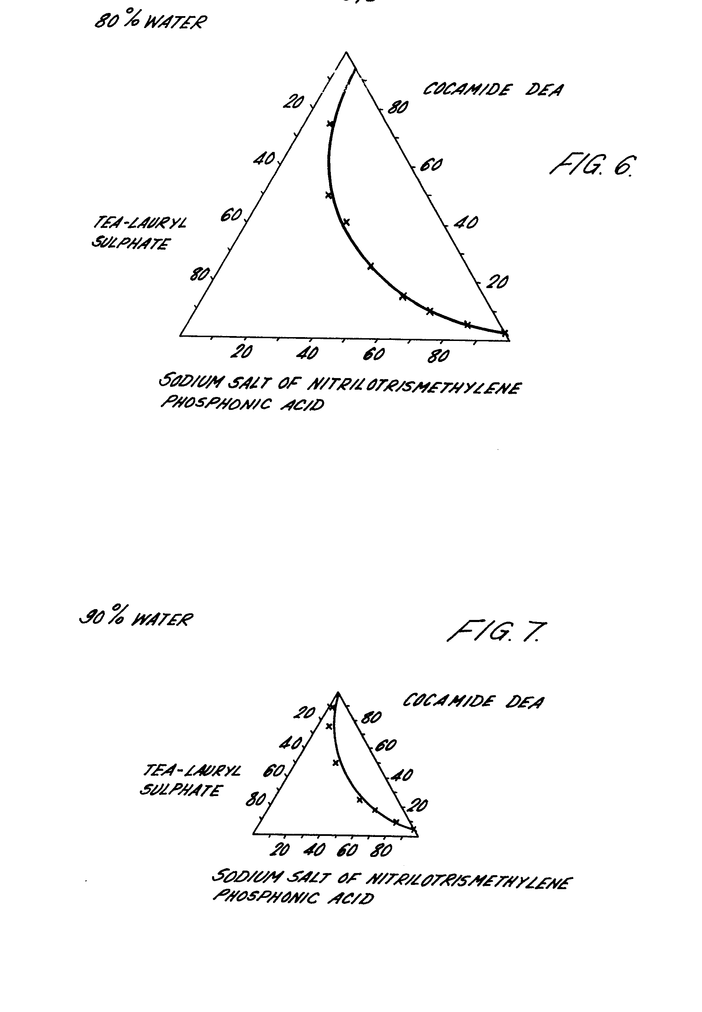

Figures 5, 6 and 7 are plots of different mixtures of as anionic surfactant, sodium laureth sulphate (sodium (2 mole ethoxylated)lauryl sulphate); as other surfactant the nonionic surfactant cocoamide DEA (coconut diethanolamide) and as sequestering agent trisodium HEDTA (the trisodium salt of N-hydroxyethyl ethylenediamine triacetic acid) respectively at 72%, 80% and 90% water contents.

A similarly shaped boundary surface is apparent from this collection of phase diagrams. Multiphase compositions lie to the right of the boundary curves and single phase compositions to the left thereof.

It will be appreciated that the compositions within the invention referred to above as falling within the boundary curves consist of the main components of the present

invention and do not include the additaments characteristic of commercial preparations.

With increase in temperature it has been found that the phase volume ratio for a given two phase composition is reduced, that is the upper layer effectively tends to diminish. Generally the multiphase compositions of the present invention when subjected to cyclic temperature changes, that is increase in temperature followed by decrease in temperature or vice versa, undergo reversible changes in phase volume ratio.

Various materials may be added to the surfactant blends as viscosity modifiers and/or clarifying agents etc. Minimal amounts of lower aliphatic alcohols, significantly below 2% by weight of the total composition are admissable in the compositions of the present invention, for example to assist in the dispersion of cosmetic components such as perfumes and other additaments of an oily nature. The various materials include conventional thickeners such as the cellulose derivatives, for example hydroxyethyl cellulose, ethylhydroxyethyl cellulose, hydroxypropylcellulose, hydroxypropylmethyl cellulose and sodium carboxymethyl cellulose; natural gums such as xanthan gum; synthetic materials such as the polyalkylene glycol block polymers and copolymers exemplified by polyethylene glycols block polymers of mean molecular weight 200 to 35000, polypropylene glycol block polymers of mean molecular weight 200 to 35000 and mixed polyethylene polypropylene glycol block copolymers of mean molecular weight less than 10,000; mildly anionic polymeric materials such as the sodium salt

of acrylic acid copolymers; higher fatty alcohols such as lauryl alcohol, cetyl alcohol and the like; as well as salts such as magnesium aluminium silicate, sodium and/or potassium citrates, sodium chloride or mixtures thereof.

A buffer may be included in the compositions of the invention to achieve a desired pH-value in the final composition. Generally the pH of the compositions lies in the range 4.5 to 10; preferably 6 to 8. It has to be observed that the phase volume ratio can vary with pH insofar as it is necessary to include a buffer to arrive at an acceptable final pH value and such inclusion changes the composition hence its location on a phase diagram.

The compositions of the present invention are nominally liquid cleaners. Users are accustomed to a degree of thickness or body in such compositions for use in the shower, the bath or as hair shampoos. Such thickness at least in part is reflected in the viscosity of a composition. The viscosity of the composition of the invention containing the main components but without customary additives varied considerably from composition to composition depending on the precise components used, some may require thickening and some may require thinning.

The range of viscosity of compositions of the present invention is up to 3000 centipoise as determined using a Brookfield viscometer model RVT using spindle No. 3 at speed 20 at 20°C. Preferably, the viscosity range is 400-1500 centipoise.

The compositions of the present invention may include the customary cosmetic additaments, for example colourants which usually affect the layers differently; perfumes; opacifying agents and pearlescing agents such as ethylene glycol monostearate; lanolin and its derivatives, preservatives, protein and hydrolysates thereof; antidandruff agents, such as coal tar solution and piroctone olamine (for example OCTOPIROX, Trade Mark of Hoechst GmbH for the monoethanolamine salt of 1-hydroxy-4-methyl-6-(2,4,4-trimethylpentyl)-2(1H)-pyridinone). It is worth remembering that the inclusion of these additaments affects the constitution of the composition of main components and hence its position on the phase diagrams, so some care has to be exercised to attain a given ratio of upper to lower layers.

In the following Examples illustrating the invention percentages are by weight of the total final composition except where otherwise indicated. Throughout, the phase volume ratios expressed are as upper:lower layers.

The method of preparing the compositions was the same throughout involving mixing together the surfactant materials, water and colourants with stirring whilst gently heating until a homogeneous mixture is formed. The sequestering agent was then added with gentle heating if necessary and the whole was allowed to cool. Finally perfume was added and the pH was adjusted with citric acid as necessary.

Example 1

| | %w/w |
|---|---|
| Sodium lauryl ether (1) sulphate (70% active) | 10.9 |
| Sodium $C_{14-16}$ olefin sulphonate (35% active) | 8.5 |
| Sodium salt of nitrilotris(methylene phosphonic acid) | 9.04 |
| Ethoxylated (4.5) coconut monoethanolamide | 6.0 |
| Coconut diethanolamide | 2.0 |
| Ethoxylated castor oil derivative (PEG 40 Castor Oil) | 2.0 |
| *Cocoamphocarboxyglycinate | 1.7 |
| *Complex N-cocamine carboxylic acid of molecular weight between 400 and 800 | 0.3 |
| (*As 2.0% w/w AMPHOLAK (Trade Mark) XCG/3 ex Amphoterics International) | |
| Perfume | qs |
| Colour | qs |
| Water | Balance to 100% |
| pH | 7.4 |

After standing, the composition exhibited two liquid phases in the volume ratio 70:30. Upon overnight storage at the temperatures indicated the following volume ratios were exhibited:-

| Storage temperature | |
|---|---|
| 0°C | 70:30 |
| 22°C | 70:30 |
| 37°C | 66:34 |
| 50°C | 63:37 |

After daily shaking the samples on 32 occasions the volume ratios were little changed viz:

| | |
|---|---|
| 0°C | 73:27 |

| | |
|---|---|
| 22°C | 71:29 |
| 37°C | 67:33 |
| 50°C | 62:38 |

Illustrating a degree of temperature stability.

Salon evaluation

When evaluated by a professional hairdresser the formulation was found to give a high volume of stable, rich, creamy lather which was easy to rinse from the hair.

When dry, the hair was easy to comb, had good body making it very manageable and smooth to the touch and had excellent gloss.

| Example 2 | %w/w |
|---|---|
| Triethanolamine lauryl ether (2) sulphate (30% active) | 25.5 |
| Sodium $C_{14-16}$ olefin sulphonate (35% active) | 8.5 |
| Sodium salt of nitrilotriacetic acid | 12.54 |
| Ethoxylated (4.5) coconut monoethanolamide | 6.0 |
| Coconut diethanolamide | 2.0 |
| Polyoxyethylene (20) glycerol monooleate | 2.0 |
| Citric acid | qs to pH 7.9 |
| Colour | qs |
| Perfume | qs |
| Water | Balance to 100% |

After standing, the composition exhibited two liquid phases in the volume ratio 67:33.

Upon storage overnight at the temperatures indicated the following volume ratios were exhibited:

| Storage temperature | |
|---|---|
| 0°C | 69:31 |
| 22°C | 65:35 |

| | |
|---|---|
| 37°C | 57:43 |
| 50°C | 49:51 |

After daily shaking the samples 33 times the volume ratios were similar to their initial values viz:

| | |
|---|---|
| 0°C | 69:31 |
| 22°C | 67:33 |
| 37°C | 59:41 |
| 50°C | 56:44 |

Salon evaluation

The formulation was evaluated in a blind trial by a professional hairdresser. It gave a high volume of a rich creamy lather, easy to rinse from the hair leaving it easy to comb.

When dry, the hair felt soft, had a good gloss and was easy to comb. It had good body, making the hair very manageable and easy to style.

Example 3

| | %w/w |
|---|---|
| Sodium lauryl ether (1) sulphate 70% active | 10.9 |
| Sodium $C_{14-16}$ olefin sulphonate (35% active) | 8.5 |
| Trisodium salt of n-hydroxyethylethylenediamine-triacetic acid | 13.76 |
| Polyoxyethylene propylene glycol dioleate | 2.0 |
| Ethoxylated (4.5) coconut monoethanolamide | 6.0 |
| Coconut diethanolamide | 2.0 |
| Colour | qs |
| Perfume | qs |
| Citric acid | qs to pH 8.6 |
| Water | Balance to 100% |

 

After standing the composition exhibited two liquid phases in the volume ratio 59:41.

Upon overnight storage at the temperatures indicated the following volume ratios were exhibited

<u>Storage temperature</u>

| | |
|---|---|
| 0°C | 59:41 |
| 22°C | 59:41 |
| 37°C | 54:46 |
| 50°C | 49:51 |

After daily shaking the samples 6 times the volume ratios were very similar to their initial volumes viz:

| | |
|---|---|
| 0°C | 64:36 |
| 22°C | 61:39 |
| 37°C | 54:46 |
| 50°C | 50:50 |

The samples were allowed to stand for 12 weeks and were then reshaken whereafter the volume ratios were found to be:-

Allowed to stand 12 weeks

| | |
|---|---|
| 0°C | 60:40 |
| 22°C | 55:45 |
| 37°C | 56:44 |
| 50°C | 51:49 |

<u>Salon evaluation</u>

Evaluation of the formulation by a professional hairdresser revealed that the product gave a rich, creamy, stable foam which was easy to rinse from the hair, leaving it easy to comb.

When dry the hair felt soft and had excellent gloss. It was easy to style, with good body and overall very manageable.

Example 4

| | % w/w |
|---|---|
| *Cocoamphocarboxyglycinate | 21.67 |
| *Complex N-cocamine carboxylic acid of molecular weight between 400 and 800 | 3.83 |
| (*As 25.5% w/w AMPHOLAK (Trade Mark) XCG/3 ex Amphoterics International) | |
| Sodium $C_{14-16}$ olefin sulphonate (35% actives) | 8.5 |
| Coconut diethanolamide | 5.0 |
| Polyethylene glycol 6000 distearate | 8.0 |
| Sodium salt of nitrilotris (methylene phosphonic acid) | 4.9 |
| Colourants | qs |
| Perfume | qs |
| water | Balance to 100% |

The product had pH = 7.98 and exhibited 3 phases in the volume ratios 34:14:52 after centrifuging at 6000 rpm for 15 minutes. The shaken product was rather viscous.

Salon evaluation

When evaluated by a professional hairdresser the formulation was found to give a high volume of stable rich creamy lather when a slightly above average amount of water was used to wet the head.

In its effect, the product compared favourably with a two-phase hair shampoo currently popular in the market place, imparting excellent cleanliness body and gloss to the dried heads which were low in static making them very

manageable and smooth to the touch.

Example 5

| | % w/w |
|---|---|
| Trisodium salt of n-hydroxyethylenediamine-triacetic acid | 3.64 |
| Cocoamide DEA | 23.20 |
| Perfume | 0.35 |
| Patent Blue V (0.1% active) | 0.30 |
| Water | Balance to 100% |

The pH of the composition was adjusted to 7.15 with citric acid.

The products exhibited two distinct layers in the volume ratio 45:55, the upper layer was opaque.

Salon evaluation

When evaluated by a professional hairdresser, the product was found to be virtually non-foaming. Rinseability was good, leaving the hair feeling "squeaky" clean and very easy to comb.

When dry, the hair was soft to the touch, had a good gloss and was very easy to manage being easy to comb and having good body.

Example 6

| | % w/w |
|---|---|
| Sodium salt of nitrilotris (methylene phosphonic acid) | 2.00 |
| Cocoamide DEA | 38.00 |
| Perfume | 0.35 |
| Patent Blue V (0.1% active) | 0.30 |
| Water | Balance to 100% |

The pH of the composition was adjusted to 7.79 with citric acid.

The product exhibited two distinct layers in the volume ratio 66:34, the upper layer was opaque.

Salon evaluation

When evaluated by a professional hairdresser, the product was found to produce a copious amount of stable, rich, creamy lather which rinsed well from the hair and left it easy to comb.

When dry, the hair felt soft, had a good gloss and was easy to manage.

The following Table illustrates additional compositions in accordance with the present invention in which the various ingredients are listed as components B to Z and AB to AN together with an amount of water sufficient to make 100% allowing for perfumes, colourants and preservatives of which a sufficiency is added, usually a relatively low concentration of each is required.

All of the compositions were liquid with two distinct layers and the phase volume ratio for each is indicated in the Table.

TABLE

Example N° ⟶

A = Phase volume ratio
B = Sodium lauryl ether (2) sulphate
C = Sodium lauryl ether (3) sulphate
D = Triethanolamine lauryl sulphate
E = Monoethanolamine lauryl sulphate
F = Ammonium lauryl sulphate
G = Triethanolamine lauryl ether (2) sulphate
H = Sodium $C_{14-16}$ olefin sulphonate
I = Sodium trideceth-7 carboxylate
J = Cocoamphocarboxyglycinate
K = Disodium pareth - 25 sulphosuccinate
L = Ethoxylated glyceryl monococoate
M = Triethanolamine salt of arylamide polyglycol ether sulphate
N = Sodium chloride
O = Sodium diethylenetriamine pentakis (methylene phosphonate)
P = Sodium salt of nitrilotris (methylene phosphonic acid)
Q = Sodium sesquicarbonate
R = Sodium heptonate
S = Trisodium salt of n-hydroxyethyl ethylenediamine triacetic acid
T = Coconut monoethanolamide ethoxylate
U = Coconut diethanolamide
V = Lauric isopropanolamide
W = Alkyl amidopropyldimethylamine betaine
X = Myristyl dimethyl amine oxide
Y = Cocamidopropyl hydroxysultaine
Z = Polyethylene glycol 6000 distearate
AB= Polyethylene glycol 4000 distearate
AC= Polyethylene glycol 120 methyl glucose dioleate
AD= Cetylalcohol polyether phosphate
AE= Quaternium 70/Propylene glycol
AF= Hydroxypropyl methyl cellulose
AG= Sodium salt of acrylic acid copolymer
AH= Hydrolysed protein
AI= Sodium deceth 7-carboxylate
AJ= Complex N-cocoamine carboxylic acid of molecular weight between 400 and 800
AK= Lauryl/myristyl dimethyl betaine
AL= Piroctone olamine
AM= Polyquaternium-7
AN= Cocoamphoglycinate
Water ad 100% and Perfume, Colourant, Preservative q.s.

TABLE (cont)

| | Example N° (%Actives) | 7 | 8 | 9 | 10 | 11 | 12 |
|---|---|---|---|---|---|---|---|
| A | | 72:28 | 64:36 | 67:33 | 66:34 | 67:33 | 75:25 |
| B | 27.5 | | | | | | |
| C | 27 | 25.5 | | | | | |
| D | 41 | | | 16.9 | 16.0 | 16.0 | 25.0 |
| E | 33 | | | | | | |
| F | 30 | | | | | | |
| G | 30 | | | | | | |
| H | 35 | 8.5 | 7.9 | 3.62 | | | |
| I | 75 | | | | | | 5.5 |
| J | 30 | | | | | | |
| K | 40 | | | | | | |
| L | 100 | | | | 4.5 | 4.5 | |
| M | 30 | | 17.7 | | | | |
| N | 100 | | | | | | |
| O | 100 | | | | | | |
| P | 100 | 6.97 | 8.2 | | 4.92 | 4.92 | 4.92 |
| Q | 100 | 5.34 | | | | | |
| R | 100 | | 3.75 | 4.0 | | | |
| S | 100 | | | 6.88 | | | |
| T | 100 | 6.0 | | | | | |
| U | 100 | 2.0 | | 7.5 | 3.0 | 3.0 | 7.0 |
| V | 100 | | | | 1.0 | | 1.5 |
| W | 30 | | 10.0 | | | | |
| X | 30 | | | | | 5.0 | |
| Y | 45 | | | | | | |
| Z | 100 | | | | 0.175 | | 2.5 |
| AB | 100 | | | | | | |
| AC | 100 | | | | | | |
| AD | 100 | | | | | | |
| AE | 54 | | | | | 1.0 | |
| AF | 100 | | | | | | 0.4 |
| AG | 100 | | | | | | |
| AH | 100 | | | | 0.7 | | |
| AI | 70 | | | | | | |
| AJ | 30 | | | | | | |
| AK | 30 | | | | | | |
| AL | 100 | | | | | | |
| AM | 8 | | | | | | |
| AN | 30 | | | | | | |

Water ad 100% and Perfume, Colourant, Preservative q.s.

TABLE (cont.)

| | Example N° (%Actives) | 13 | 14 | 15 | 16 | 17 | 18 |
|---|---|---|---|---|---|---|---|
| A | | 65:35 | 74:26 | 84:16 | 77:23 | 47:53 | 75:25 |
| B | 27.5 | | | | | | 42.73 |
| C | 27 | | | | | | |
| D | 41 | 26.4 | | | | | |
| E | 33 | | 32.0 | | | | |
| F | 30 | | | | 35.3 | | |
| G | 30 | | | | | 25.5 | |
| H | 35 | | | | | 8.5 | |
| I | 75 | | | | | | |
| J | 30 | | | | | | |
| K | 40 | | | | | | |
| L | 100 | | | | | | |
| M | 30 | | | | | | |
| N | 100 | 2.0 | | | | | |
| O | 100 | | | | | | |
| P | 100 | 4.1 | 4.1 | 4.1 | 3.28 | | 4.92 |
| Q | 100 | | | | | | |
| R | 100 | | | | | | |
| S | 100 | | | | | 13.76 | |
| T | 100 | 5.0 | | | | 6.0 | |
| U | 100 | | 2.0 | 5.0 | 2.0 | 2.0 | 5.0 |
| V | 100 | | | | | | |
| W | 30 | | | | | | |
| X | 30 | | | | | | |
| Y | 45 | | | | | | |
| Z | 100 | 8.0 | | 8.0 | 8.0 | | 8.0 |
| AB | 100 | | 8.0 | | | | |
| AC | 100 | | | | | 2.0 | |
| AD | 100 | | | | | | |
| AE | 54 | | | | | | |
| AF | 100 | | | | | | |
| AG | 100 | | | | | | |
| AH | 100 | | | | | | |
| AI | 70 | | | | | | |
| AJ | 30 | | | | | | |
| AK | 30 | | | | | | |
| AL | 100 | | | | | | |
| AM | 8 | | | | | | |
| AN | 30 | | | 42.7 | | | |

Water ad 100% and Perfume, Colourant, Preservative q.s.

TABLE (cont.)

| | Example N° (%Actives) | 19 | 20 | 21 | 22 | 23 | 24 | 25 |
|---|---|---|---|---|---|---|---|---|
| A | | 96:4 | 43:57 | 67:33 | 62:38 | 73:27 | 58:42 | 54:46 |
| B | 27.5 | | | 18.0 | | | | |
| C | 27 | | | | | 25.5 | | |
| D | 41 | | | | | | | |
| E | 33 | | | | 32.0 | | | |
| F | 30 | | | | | | | |
| G | 30 | | | | | | | |
| H | 35 | 33.57 | | | | 8.5 | | |
| I | 75 | | | | | | | |
| J | 30 | | | | | | 7.4 | 7.4 |
| K | 40 | | 30.0 | | | | | |
| L | 100 | | | 4.0 | | | | |
| M | 30 | | | | | | | |
| N | 100 | | | | | | | |
| O | 100 | | | | 4.89 | | | |
| P | 100 | 4.92 | 4.92 | 4.92 | | 8.2 | 5.74 | 5.74 |
| Q | 100 | | | | | | | |
| R | 100 | | | | | | | |
| S | 100 | | | | | | | |
| T | 100 | | | | | | | |
| U | 100 | 5.0 | 2.0 | 5.0 | | 5.0 | | |
| V | 100 | | | | | | | |
| W | 30 | | | | | | | |
| X | 30 | | | | | | | |
| Y | 45 | | | 5.67 | | | | |
| Z | 100 | 8.0 | 8.0 | | 8.0 | | | |
| AB | 100 | | | | | | | |
| AC | 100 | | | | | 1.5 | 3.0 | 3.0 |
| AD | 100 | | | 5.0 | | | | |
| AE | 54 | | | 1.0 | | | | |
| AF | 100 | | | | | | | |
| AG | 100 | | | 0.3 | | | | |
| AH | 100 | | | | | | | |
| AI | 70 | | | | | | 11.57 | 11.57 |
| AJ | 30 | | | | | | 1.6 | 1.6 |
| AK | 30 | | | | | | 5.0 | 5.0 |
| AL | 100 | | | | | | 0.7 | |
| AM | 8 | | | | | | 3.0 | 3.0 |
| AN | 30 | | | 2.0 | | | | |

Water ad 100% and Perfume, Colourant, Preservative q.s.

The product of Example 24 was subject to storage shaking tests and limited salon evaluation.

The pH of the final composition was 7.49 and viscosity of the mixture was measured as 90 cps. After standing, the product exhibited two distinct layers in the volume ratio 58:42, the upper layer was translucent.

Upon overnight storage at the temperatures indicated the following volume ratios were exhibited:-

| Storage temperature | |
|---|---|
| 0°C | 56:44 |
| 22°C | 57:43 |
| 37°C | 57:43 |
| 50°C | 55:45 |

After shaking samples once daily for each of 31 successive days the volume ratios were:-

| | |
|---|---|
| 0°C | 57:43 |
| 22°C | 57:43 |
| 37°C | 57:43 |
| 50°C | 57:43 |

and the upper phase remained translucent.

When evaluated by a professional hairdresser on five heads the product was found on wet assessment to be satisfactory for greasy hair and better on normal to dry hair. On dry assessment the hair was left with excellent body with good gloss and ease of combing.

For the product of Example 25 the pH of the final composition was 7.3 and the viscosity of the mixture was measured as 50 cps. After standing, the product exhibited two distinct layers in the volume ratio 54:46, the upper

layer was translucent with a beautiful sky blue colour.

As a hair shampoo the product behaved as the product of Example 25 without an antidandruff feature.

In this specification some detergents are named in accordance with the "CTFA Cosmetic Ingredient Dictionary" published 1982 by The Cosmetic, Toiletry and Fragrance Association, Inc. at Washington, D.C., U.S.A.

CLAIMS

1. A liquid cleansing composition comprising water; at least one surfactant; and at least one sequestering agent, the relative concentrations of the components being so chosen that the composition has at least two distinct layers which are temporarily interdispersible upon agitation and wherein each layer is miscible with water in substantially all proportions.

2. A composition as claimed in Claim 1 in which the surfactant comprises at least one anionic, amphoteric or nonionic surfactant or a mixture thereof.

3. A composition as claimed in Claim 1 or Claim 2 in which the surfactant comprises at least one anionic surfactant and at least one surfactant other than an anionic surfactant.

4. A composition as claimed in Claim 1 or Claim 2 in which the concentration of surfactant comprises up to 50% by weight of the composition.

5. A composition as claimed in Claim 1 or Claim 2 in which the sequestering agent comprises up to 30% by weight of the composition.

6. A composition as claimed in any preceding claim in which the sequestering agent provides a complexing anion which is strong enough for a 0.01M solution thereof to be in equilibrium with less than $10^{-3}$ mole of calcium ion.

7. A composition as claimed in any preceding claim in which the sequestering agent comprises at least one compound which is an organophosphonic acid or salt thereof, an aminocarboxylic acid or a salt thereof or a mixture thereof.

8. A composition as claimed in Claim 7 which includes a polyhydroxycarboxylic acid or salt thereof and/or sodium sesquicarbonate.

9. A composition as claimed in any preceding Claim in which the sequestering agent comprises at least one of:-

(i) organophosphonates of general formula (I)

$$R^1\text{-N-}(R)_2 \qquad (I)$$

wherein $R = -CH_2PO_3M_2$, M represents hydrogen, or a cosmetically acceptable organic or inorganic base; and $R^1$ is a straight or branched chain alkyl group which may contain an odd or even number of carbon atoms up to 14 in number, a hydroxyalkyl group containing from 2 to 6 carbon atoms, or a group

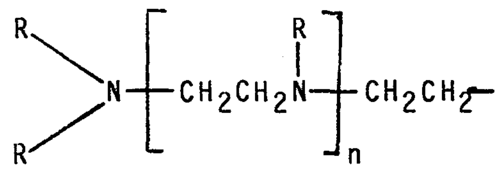

in which n = 0, 1, 2, 3 or 4; or $R^1$ = R: or

(ii) aminocarboxylic acids of general formula (II)

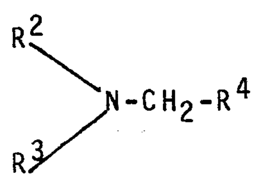 (II)

wherein $R^2$ = H, $-CH_2CH_2OH$ or $-CH_2COOM$;

$R^3$ = $-CH_2CH_2OH$, $-CH_2COOM$ or 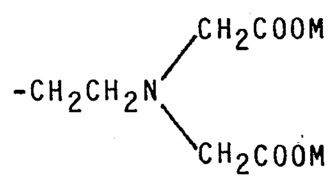;

$R^4$ = $-COOM$ or 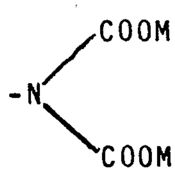

where M is as defined above: or mixtures thereof.

10. A composition as claimed in any preceding claim including a polyhydroxycarboxylic acid and containing 5 to 7 carbon atoms, optionally in a ring, and more than two hydroxyl groups per molecule, salts thereof with cosmetically acceptable organic and/or inorganic basis, and/or sodium sesquicarbonate.

11. A composition as claimed in Claim 5 in which the sequestering agent comprises up to 25% by weight of the composition.

12. A composition as claimed in Claim 11 in which the sequestering agent comprises up to 15% by weight of the composition.

13. A composition as claimed in Claim 11 in which the sequestering agent comprises from 2% to 15% by weight of the composition.

14. A composition as claimed in any preceding claim in which the anionic surfactant is a blend of anionic surface active agents.

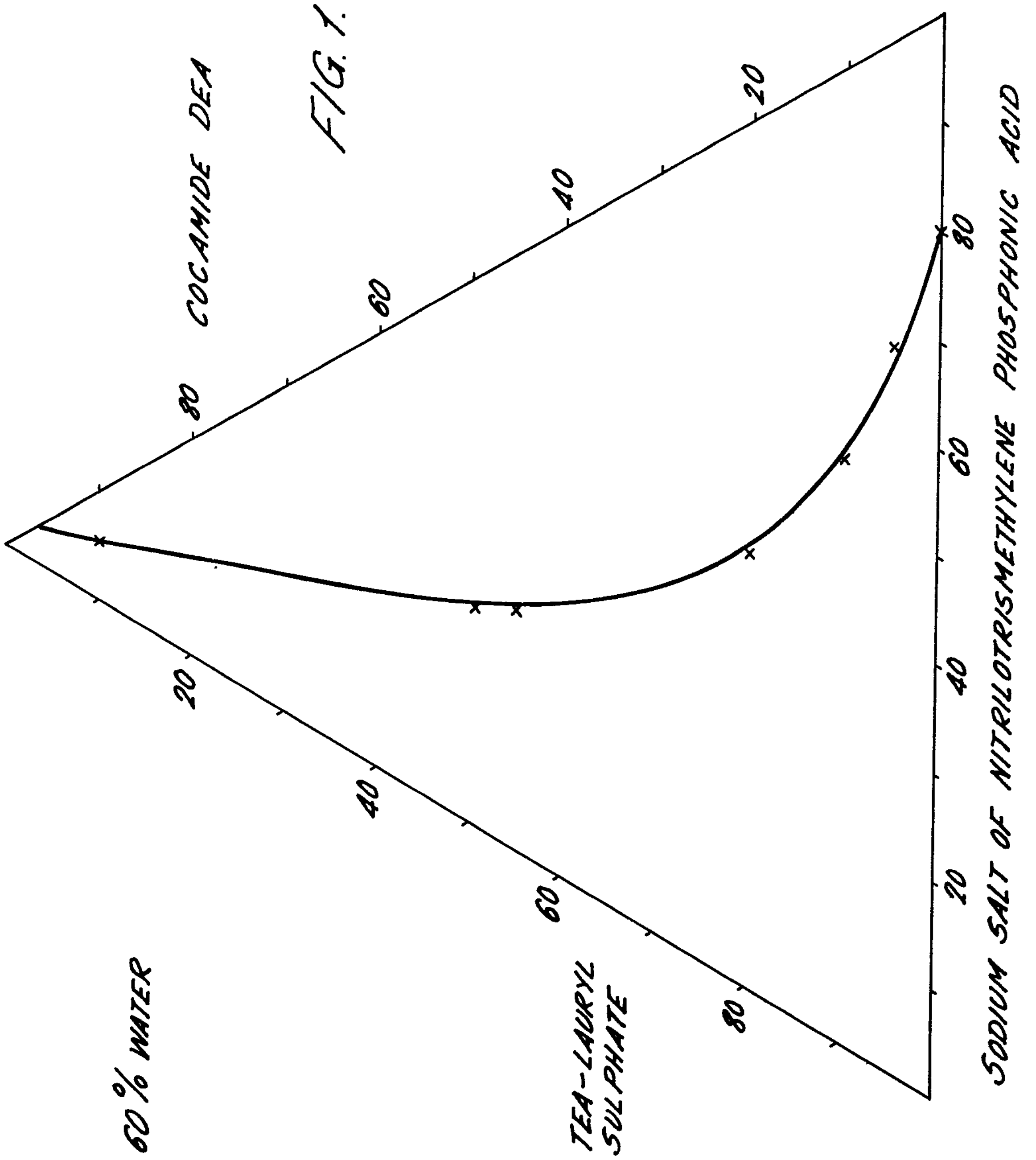

COCAMIDE DEA
60% WATER
TEA-LAURYL SULPHATE
SODIUM SALT OF NITRILOTRISMETHYLENE PHOSPHONIC ACID
20
40
60
80


FIG. 1.

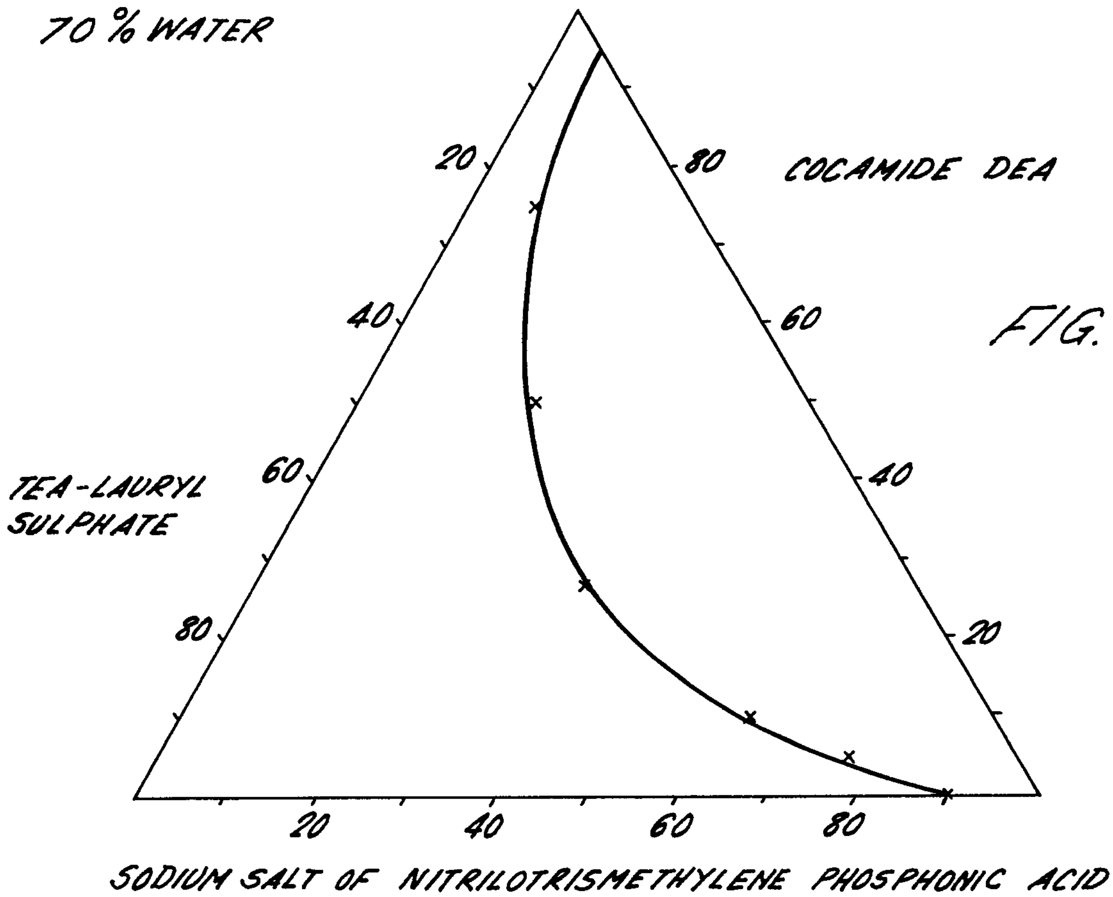
70 % WATER
20
40
60
80
TEA-LAURYL SULPHATE
80
60
40
20
COCAMIDE DEA
20
40
60
80
SODIUM SALT OF NITRILOTRISMETHYLENE PHOSPHONIC ACID

FIG. 2.

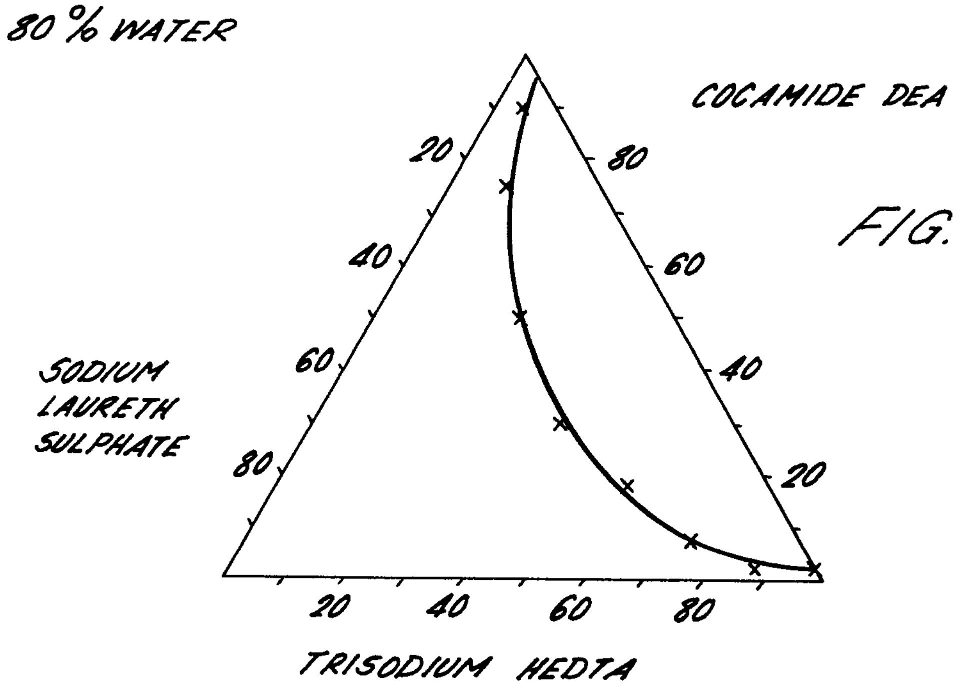
80 % WATER
COCAMIDE DEA
20
40
60
80
80
60
40
20
SODIUM
LAURETH
SULPHATE
20 40 60 80
TRISODIUM HEDTA

FIG. 3.

FIG. 4.

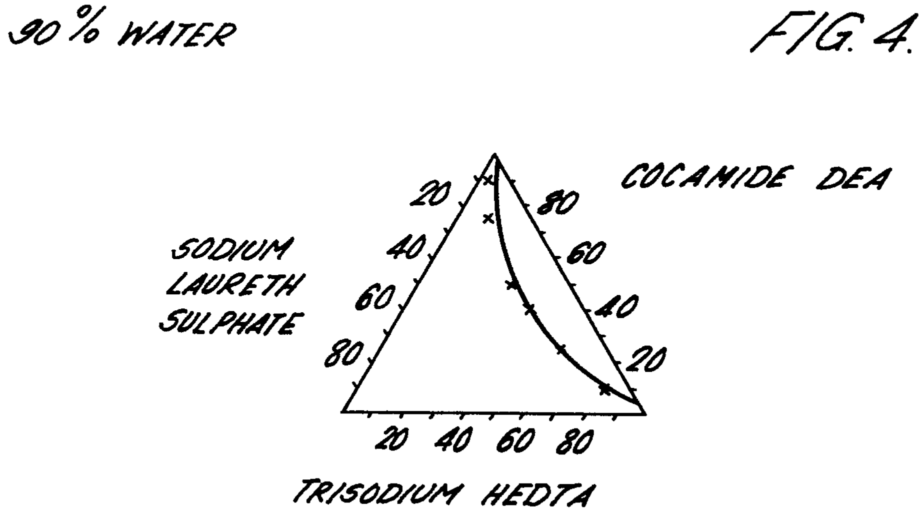
90 % WATER
COCAMIDE DEA
20
40
60
80
80
60
40
20
SODIUM
LAURETH
SULPHATE
20 40 60 80
TRISODIUM HEDTA

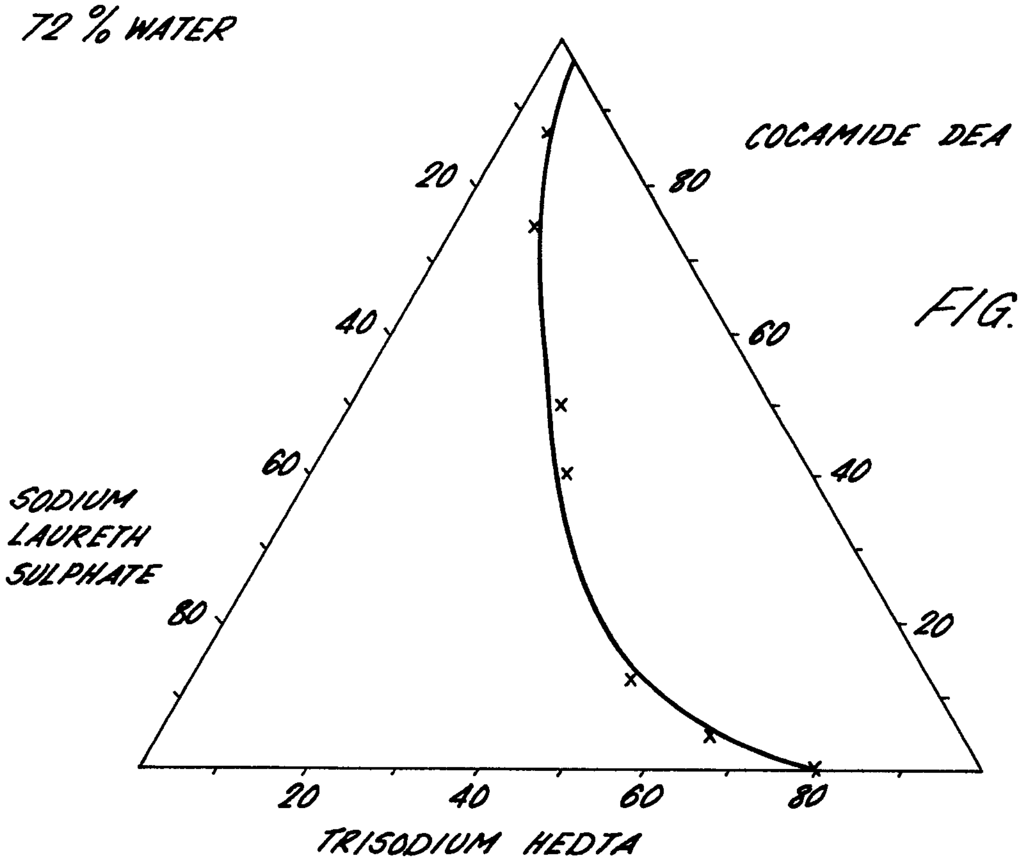
72 % WATER
COCAMIDE DEA
SODIUM LAURETH SULPHATE
TRISODIUM HEDTA
20
40
60
80
80
60
40
20
20
40
60
80

FIG. 5.

0175485

5/5

80 % WATER

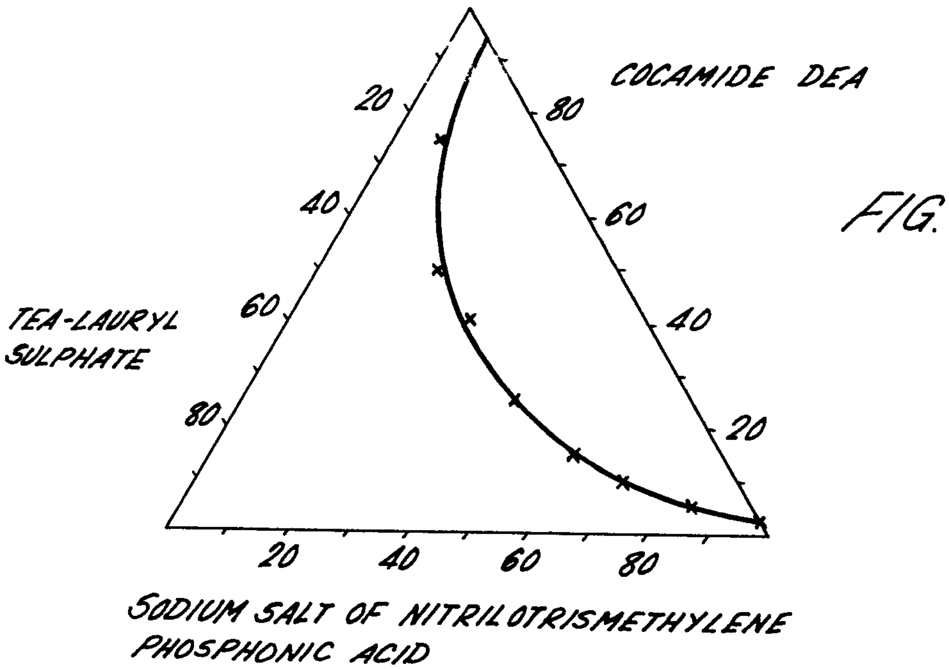


FIG. 6.

90 % WATER

FIG. 7.